# EUROPÄISCHE PATENTSCHRIFT

(11) **EP 3 072 553 B1**
(45) Veröffentlichungstag und Bekanntmachung des Hinweises auf die Patenterteilung: **23.08.2017**
(21) Anmeldenummer: 16157264.9
(22) Anmeldetag: 25.02.2016
(51) Int. Cl.: A61N 1/375, H01R 13/03, H01G 4/35

(54) **ANSCHLUSSSTIFT UND DURCHFÜHRUNG**
TERMINAL PIN AND FEEDTHROUGH
BROCHE DE RACCORDEMENT ET TRAVERSÉE

(30) Priorität: 20.03.2015 US 201562135710 P
(43) Veröffentlichungstag der Anmeldung: 28.09.2016
(73) Patentinhaber: BIOTRONIK SE & Co. KG, 12359 Berlin (DE)
(72) Erfinder: Pscherer, Norbert, 90766 Fürth (DE); Kronmüller, Daniel, 90409 Nürnberg (DE); Teske, Josef, 96103 Hallstadt (DE)
(74) Vertreter: Galander, Marcus

(56) Entgegenhaltungen:
- EP-A1- 1 695 736
- US-A1- 2011 303 458
- US-A1- 2013 176 658
- US-B2- 7 340 305

## Beschreibung

Die Erfindung betrifft einen Anschlussstift zum elektrischen Anschluss eines Leitungsträgers, der zur stoffschlüssigen Anbindung an eine Leiterfläche des Leitungsträgers vorgesehen ist. Sie betrifft des Weiteren eine Durchführung eines implantierbaren elektromedizinischen Gerätes (IMD) sowie schließlich ein solches Gerät insgesamt.

Die meisten praktisch bedeutsamen implantierbaren elektromedizinischen Geräte (IMDs) sind dazu vorgesehen, über geeignet platzierte Elektroden elektrische Impulse an reizbares Körpergewebe abzugeben. Ferner können viele Geräte elektrische Impulse und Reize im Körper des Patienten gezielt messen und über einen längeren Zeitraum aufzeichnen oder auswerten, um eine individuell angepasste Therapie zu wählen und den Erfolg der Behandlung in vivo zu überprüfen.

Um diese Funktionen auszuführen, sind im Gehäuse des Gerätes elektronische/elektrische Funktionseinheiten zur Erzeugung und Messung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Diese Aufgabe wird von sogenannten Durchführungen übernommen, die Gegenstand zahlreicher und höchst unterschiedlicher Entwicklungen gewesen sind. Die Aufgabe einer Durchführung besteht darin, die elektrischen Signale durch das hermetisch geschlossene Gehäuse zu führen und so einen elektrischen Kontakt der Elektronik im hermetisch dichten Gehäuse mit den Elektroden im Körper des Patienten zu ermöglichen. In vielen solchen Durchführungen werden hierfür Anschlussstifte eingesetzt, die im Geräteinneren mit der dort befindlichen Leiterplatte oder einem ähnlichen Leitungsträger kontaktiert werden und die Signale durch das Gehäuse führen.

Im Zuge der Miniaturisierung und der Fortschritte der Elektronik hat sich die Weichlöttechnik (Solder) in den letzten Jahren rasant gewandelt. Manuelle Bestückungsprozesse wurden zunehmend von vollautomatischen Pick&Place-Automaten verdrängt und die Through Hole Technology (THT) wurde allmählich durch die Oberflächenmontage (SMT) ersetzt. Dies ermöglicht kleinere, kompaktere Schaltungen und führt somit schrittweise auch zu kleineren, patientenverträglicheren IMDs. Um die vielen Vorteile der SMT erreichen zu können, müssen Durchführungen die Anforderungen an ein oberflächenmontiertes Bauteil (SMD)genügen. Hierzu muss insbesondere der eingesetzte, meist in einem Hochtemperaturprozess eingelötete (brazed) Stift hohen elektrischen, thermischen und mechanischen Anforderungen genügen, bevor er an die Leiterplatte oder Leitungsträger weichverlötet (solder) werden kann. Die Zuverlässigkeit aller Fügestellen der Durchführung muss über die gesamte Lebenszeit auch unter dem Einfluss von Körperflüssigkeiten sichergestellt sein.

Es gibt daher vollständig aus einem Edelmetall (etwa Platin, Platin/Iridium, Palladium) gebildete Anschlussstifte, die leicht und zuverlässig in einem Weichlötverfahren mit dem Leitungsträger verbunden werden können, bei denen der hohe Preis aber nachteilig ist. Darüber hinaus gibt es mehrteilige Anordnungen, die etwa den eigentlichen Anschlussstift und ein weichlötbares Zusatzteil (Pad, Hülse) aus unterschiedlichen Materialien umfassen.

Die letzteren Lösungen erfordern einen gesonderten Montage- und Fertigungsschritt. Es werden Komponenten erstellt, die nach oder während dem Hochtemperaturlöten (brazing) mit der Durchführung gefügt werden. Der zusätzliche Bestückungsaufwand ist nicht unerheblich. Er kann dazu führen, dass die Fertigungskosten für die zusätzlichen Komponenten deren Materialwert um ein Vielfaches übersteigen. Die zusätzlichen Komponenten sind meist sehr klein und grazil (typischerweise <1mm) und daher schwierig zu platzieren und auszurichten. Weiterhin werden separate Fertigungshilfsmittel zur Montage der zusätzlichen Komponenten benötigt. Die Komponenten müssen vor der Montage extra gelagert und geprüft werden und benötigen zusätzliche Chargenführung und -kontrolle.

Durch den zusätzlichen Fügeprozess entsteht potenziell Ausschuss. Findet der Fügeprozess des Stiftes aus Komponenten integriert mit dem Fügeprozess der Durchführung statt, so muss eine zusätzliche Sortierprüfung durchgeprüft werden, bei der der Prozess des Stiftfügens beurteilt wird. Nach dem Fügen muss die Verbindungsstelle auf Haftung, ausreichende Stabilität und Lebensdauer untersucht werden. Grundsätzlich können eine Verkippung und ein Versatz der Bauteile des Stiftes und dadurch resultierend ein Versatz beim Platzieren auf der Leiterplatte auftreten. Dieser muss in einem gesonderten Prüfschritt festgestellt und bei der Durchführungs-Montage vermieden und geprüft werden.

Aus der US 7,340,305 B2 ist eine Durchführung eines IMD bekannt, in der Anschlussstifte eingesetzt werden, die einen nicht aus teurem Platin, Platin/Iridium oder Palladium bestehenden Kern und eine ein- oder mehrteilige leitfähige Beschichtung haben. Die Beschichtung ermöglicht es einerseits, eine Oxidation des Anschlussstifts unter den Einsatzbedingungen eines implantierten Gerätes zu steuern bzw. zu begrenzen, und andererseits ermöglicht sie es, den Anschlussstift durch ein Weichlötverfahren mit einer Leiterplatte zu verbinden. Auch die US 2011/0303458 A1 offenbart Anschlussstifte mit partiell mehrschichtigem Aufbau und deren Einsatz bei Durchführungen von IMDs.

Die US 7,747,321 B2 offenbart, wie in Fig. 1 gezeigt, einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet. Sie umfassen einen Draht-Kern, etwa aus Tantal, Niob, Titan, Molybdän oder Kupfer und eine oxidationsbeständige Ummantelung aus einem biokompatiblem Material, etwa Gold, Platin, Titan oder ähnlichem.

Aufgabe der Erfindung ist es, einen gegenüber bekannten Anordnungen verbesserten Anschlussstift bereitzustellen, der insbesondere kostengünstig herzustellen und zu verarbeiten ist und dessen Einsatz einen verringerten Prüf- und Montageaufwand bei der Realisierung der Oberflächenmontage (SMT) von Durchführungen ermöglicht.

Diese Aufgabe wird durch einen Anschlussstift mit den Merkmalen des Anspruchs 1 gelöst. Zweckmäßige Fortbildungen des Erfindungsgedankens sind Gegenstand der abhängigen Ansprüche. Mit der Erfindung wird des Weiteren eine Durchführung mit den Merkmalen des Anspruchs 16 bereitgestellt.

Erfindungsgemäß ist vorgesehen, dass der Anschlussstift an oder nahe einem Ende einen gegenüber einem end-ferneren Abschnitt verdickten Abschnitt hat und mindestens ein Teil der Oberfläche des verdickten Abschnitts eine weichlötbare Beschichtung mit einem Edelmetall aufweist. Dies bewirkt gemäß einem ersten Aspekt der Erfindung, dass der vorgeschlagene Anschlussstift auf einer gewöhnlichen SMD-Leiterplatte oder einem ähnlichen Leitungsträger mittels eines etablierten Weichlotverfahrens gefügt werden kann, wobei das Lötverfahren kompatibel zu etablierten Prozessen und Materialien ist und eine langzeitstabile Verbindung liefert.

Dies gewährleistet hohe Formstabilität und Reproduzierbarkeit bei der Herstellung des Anschlusses und/oder ermöglicht - gemäß einem zweiten, relativ unabhängigen Aspekt der Erfindung - die Realisierung einer diffusionshemmenden Engstelle im Längsverlauf des Anschlussstiftes und somit im Bereich einer mit einem solchen Stift gebildeten Durchführung.

Gegenüber etablierten Konfigurationen, die mehrteilige Anschlussstifte beinhalten, ergeben sich folgende spezielle Vorteile:
- Integration mehrerer Komponenten in eine Komponente
- Kostengünstigere Produktion ohne Redesign der Produkte bzw. Produktneuentwicklung
- Geringere Ausschusszahlen
- Weniger Bestückungsaufwand mit der Folge niedrigerer Kosten
- Höhere Maß und Positionsgenauigkeit.

In einer Ausführung der Erfindung ist der verdickte Abschnitt rotationssymmetrisch am Ende des Anschlussstiftes im Wesentlichen in Art eines Nagelkopfes gebildet. Diese Ausführung ist mit seit langem etablierten Stauchverfahren sehr leicht und kostengünstig herzustellen und bietet, je nach konkreter Ausführung, die Möglichkeit einer besonders einfachen Anpassung der Endfläche des Anschlussstiftes an die für die Verbindung mit der entsprechenden Leiterbahn verfügbare Grundfläche.

In einer weiteren Ausgestaltung der Erfindung wird die Formgebung am Ende des Stiftes der Kontaktfläche (Land Pattern) der Leiterplatte (PCB) angepasst. Bekannte und häufig verwendete Grundflächen sind Rechtecke, Sechsecke und Achtecke oder andere Polygone. Um die leichte Herstellbarkeit gewährleisten zu können, müssen die Ecken des Umformwerkzeuges verrundet werden, damit der Stift nach dem Umformen ausgeformt werden kann. Die Kontaktfläche zwischen Durchführungsstift und Leiterplatte ist vergrößert, wodurch der Übergangswiderstand gesenkt und die Belastbarkeit der Fügeverbindung zwischen Stift und Platine vergrößert werden können.

In einer Ausgestaltung dieser Ausführung ist am äußersten Ende des Anschlussstiftes, ein insbesondere kegelförmiger, halbkugelförmiger oder zylindrischer Spitzenabschnitt ausgeformt. Vorteilhaft ist eine solche zusätzliche Geometrie an der Unterseite des Stiftes, welche die Funktion einer Auflagefläche auf der Platine übernimmt, aus den folgenden Gründen: Der Stift darf während des Weichlötprozesses nicht vollflächig mit der Platine abschließen. Es bildet sich ein definierter Lötspalt zwischen Stift und Platine in den flüssiges Weichlot eindringen kann bzw. in dem Lötpaste beim Aufsetzen der Durchführung und der anderen der Bauteile in die Lötpaste (solder paste) nicht komplett verdrängt wird bzw. an definierten Stellen verbleibt.

Vorteilhaft kann es im Übrigen sein, die Stiftenden spitz oder scharfkantig ausführen, damit diese sich durch definiertes Andrücken im Substratmaterial der Leiterplatte (PCB) zentrieren bzw. fixieren können. So ist gewährleistet, dass die Durchführung während des Durchfahrens im Reflowofens in der Lötpaste nicht aufschwimmt oder verrutscht. Lötfehler wie Seiten- bzw. Spitzenüberhang können so effektiv vermindert werden. Der Prozess kann hierdurch mit üblichen Prozessparametern ablaufen, auch wenn der Masseschwerpunkt bzw. das Trägheitsmoment der Durchführung designbedingt untypisch für SMD-Bauteile liegen.

In einer Ausführung gemäß dem zweiten Aspekt der Erfindung ist der verdickte Abschnitt bezüglich eines end-ferneren Abschnitts definiert, welcher einen gegenüber dem Durchmesser, den der Anschlussstift im größeren Teil seiner Längserstreckung hat, verringerten Durchmesser hat. Insbesondere weist der, auf den end-ferneren Abschnitt mit verringertem Durchmesser bezogen, verdickte Abschnitt den gleichen Durchmesser wie der Anschlussstift im größeren Teil seiner Längserstreckung auf. Dies erfordert lediglich die Einformung einer Engstelle im Längsverlauf des Anschlussstiftes, etwa in einem einfachen Streck- oder Walzschritt.

Die erwähnten Ausführungen sind miteinander kombinierbar; insbesondere ist in einer Ausführung der end-fernere Abschnitt mit verringertem Durchmesser unmittelbar benachbart oder mit vorgegebenem Abstand zum nagelkopf-artigen Endabschnitt des Anschlussstiftes gebildet. Hiermit lassen sich auch die Wirkungen gemäß dem ersten und zweiten Aspekt der Erfindung in synergistischer Weise kombinieren, wobei die Voreinstellung eines gewünschten Abstandes zwischen dem Nagelkopf-Ende und der Engstelle des Anschlussstiftes eine präzise Anpassung an die geometrische Konfiguration der Leiterplatte bezüglich der Durchführung eines IMD erlaubt.

Eine Vervielfachung der weiter oben erwähnten Wirkungen speziell gemäß dem zweiten Aspekt der Erfindung lässt sich in einer Ausführung erreichen, in der der Anschlussstift mehrere verdickte und beschichtete Abschnitte aufweist. Hierbei kann spezieller vorgesehen sein, dass mindestens ein Teil der mehreren verdickten und beschichteten Abschnitte mit einer zusammenhängenden Beschichtung versehen ist, die sich über dazwischenliegende Abschnitte geringeren Durchmessers erstreckt.

Generell kann die Beschichtung die gesamte Oberfläche des oder jeden verdickten Abschnitts bedecken, was eine technologisch besonders einfache Erzeugung der Beschichtung ermöglicht. Andererseits kann es zweckmäßig sein, dass die Beschichtung nur den Boden und optional den Umfang oder Teilabschnitte des Umfangs des nagelkopf-artigen verdickten Abschnitts bedeckt. Die Beschichtung kann aus einer oder mehreren dünnen Schichten bestehen, welche auch in einem Prozess übereinander aufgebracht werden können. Eine Beschichtung kann zur vorteilhaften Ausführung eines direkten Weichlötens des Anschlussstiftes mit dem Leitungsträger oder auch zur Bonden von Anschlussdrähten an den Anschlussstift führen.

In einer weiteren Ausführung der Erfindung weist der Stift mehrere Biegungen oder Rundungen längs seiner Achse auf. Die Biegung kann während eines Umformprozesses hergestellt werden. Insbesondere nach dem Fügen der Stifte zu einer Durchführung lassen sich die Stifte sehr einfach ausrichten bzw. in Form bringen. Dies ermöglicht, dass die Stifte der Durchführung das Gehäuse nicht parallel zur Leiterplatte verlassen, sondern in einem beliebigen Winkel, z.B. 90° zur Leiterplatte.

Als Material des unbehandelten Stiftes bzw. des Stift Grundkörpers kommen biokompatible leitfähige Materialen (z.B. Titan, Tantal, Niob) in Frage. Alternativ kann der Anschlussstift aus einem kostengünstigen Substrat (z.B. Kupfer, Konstantan, Nickel) gefertigt sein. In der Ausführung, dass es sich um nicht biokompatibles Material handelt, wird der Stift nach dem Umformen mit einem biokompatiblen Material (z.B. Titan, Tantal, Niob, Platin, Palladium oder Legierungen daraus) beschichtet. Die Beschichtung muss mindestens den Teil umfassen, welcher sich nicht innerhalb des späteren IMDs Gehäuse befindet.

Zum Herstellen der elektrischen Anbindung wird gemäß einer weiteren Ausführung am Stift im unmittelbaren Bereich der Verdickung eine gut weichlötbare Oberfläche freigelegt, z. B. mittels eines Flussmittels oder Vorbereiten der Oberfläche (Plasmaaktivieren oder Ätzen).

Die weichlötbare Beschichtung kann mindestens ein Edelmetall oder eines der Metalle Nickel, Kupfer, Silber, Zinn oder eine Legierung mit mindestens eines dieser Metalle enthalten. Typische Schichtdicken betragen einige Mikrometer. Es sind auch verwandte Metalle einsetzbar, so etwa eines oder mehrere der Elemente Iridium, Rhodium oder Ruthenium. Es versteht sich, dass auch verschiedenste Legierungen von mindestens zwei der hier erwähnten Elemente, vorteilhaft insbesondere kommerziell relativ kostengünstig verfügbare Legierungen, bei der Ausführung der Erfindung verwendbar sind. Als Material des unbehandelten Anschlussstiftes bzw. des Stift-Grundkörpers kommen insbesondere Kupfer oder Titan, aber auch Elemente wie Tantal, Niob oder Molybdän und auch wiederum Legierungen dieser in Betracht.

In einer weiteren Ausführung, die insbesondere im Kontext des zweiten Aspekts der Erfindung steht, ist eine direkt berührende Schicht der Beschichtung als Diffusionsbarriere ausgebildet, und diese weist insbesondere mindestens eines der Metalle Aluminium, Nickel oder Gold auf. Die Diffusionsbarriere kann über oder unter die weichlötbare Beschichtung aufgebracht werden. Es kann auch sinnvoll sein, mehrere übereinander aufgebrachte Diffusionsbarrieren zu erzeugen und diese zu kombinieren oder übereinander zu schichten, um eine besonders hohe Langlebigkeit der mit einem solchen Anschlussstift gebildeten Durchführung zu erreichen.

Gemäß einer technologischen Ausgestaltung der Erfindung ist die Beschichtung oder mindestens eine Schicht hiervon als galvanische Beschichtung oder als durch ein Vakuum-Beschichtungsverfahren erzeugte Dünnschicht ausgeführt. Zweckmäßige Schichtdicken liegen zwischen 0,1 µm (für ein Dünnschichtverfahren) und 10 µm (für ein Galvanikverfahren). Beide Ausführungen haben spezifische Vor- und Nachteile; beispielsweise ist die Schichtbelegung bei der Galvanik sehr selektiv, die Schicht jedoch häufig mit Fremdstoffen aus dem Galvanikbad kontaminiert. Das Sputtern unter Vakuum erfolgt zwar allseitig jedoch mit sehr hoher Reinheit.

Zum Schutz der Beschichtung vor Verunreinigung, Kontamination und Oxidation kann die Beschichtung auf dem Stift bis zur weiteren Verarbeitung versiegelt werden. Hierfür kann ein Polymer oder ein organischer Schutzfilm verwendet werden (OSP - Organic Surface Protection). Bekannte Schutzfilme (z.B. Glicoat, ENTEK+) können vollständig oder selektiv auf die weichlötbare Schicht oder den ganzen Stift aufgebraucht sein. Typische Schichtdicken betragen 0,2 µm bis 0,6 µm und enthalten zum Beispiel substituierte Imidiazole und/oder Triazole. Der Schutzfilm verhindert die Oxidation des Grundmaterials während der Lagerung typischerweise für mehrere Monate und pyrolysiert unmittelbar vor bzw. während Weichlötprozesses. Verbrennungsrückstände auf den Platinen können während der etablierten Waschprozesse in automatisierten Anlagen rückstandsfrei entfernt werden.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische Darstellung eines herkömmlichen Herzschrittmachers,
- Fig. 2: schematische partielle Querschnittsdarstellungen von Anschlussstiften gemäß Ausführungsformen der Erfindung,
- Fig. 3: eine partielle Querschnittsdarstellung eines Anschlussstiftes gemäß einer weiteren Ausführung der Erfindung,
- Fig. 4: schematische partielle Querschnittsdarstellungen von Anschlussstiften gemäß weiteren Ausführungsformen der Erfindung,
- Fig. 5: schematische partielle Querschnittsdarstellungen von Anschlussstiften gemäß weiteren Ausführungsformen der Erfindung,
- Fig. 6: schematische partielle Querschnittsdarstellungen von Anschlussstiften gemäß weiteren Ausführungsformen der Erfindung,

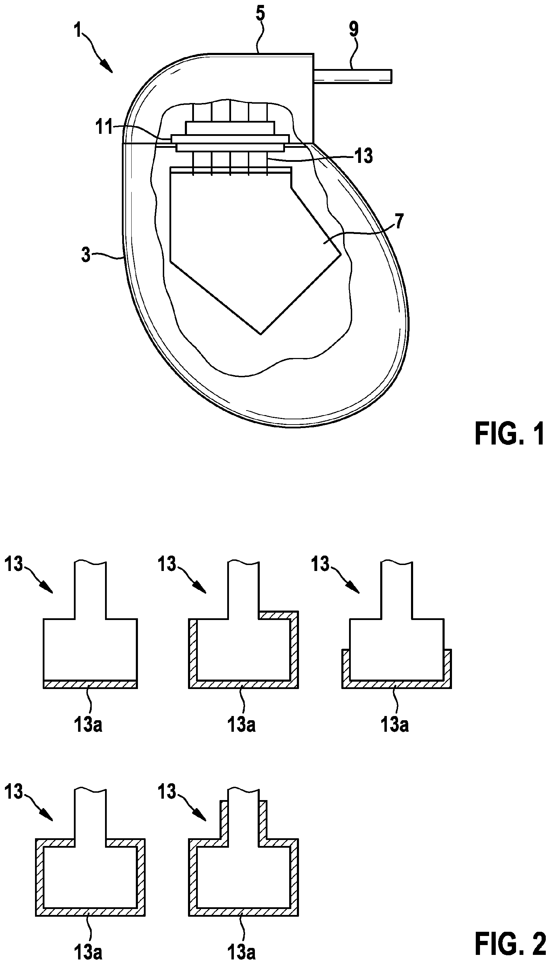

In allen Figuren 2 bis 6 ist der Anschlussstift jeweils, in Anlehnung an Fig. 1 und unabhängig von den abweichenden geometrischen Formen und Anordnungen der Beschichtung, einheitlich mit den Ziffern 13 bzw. 13' bezeichnet, und die Bezeichnung einzelner Abschnitte oder der jeweiligen Beschichtung ist entsprechend gewählt.

Fig. 2 zeigt mehrere Ausführungen eines Anschlussstiftes 13 mit nagelkopf-artigem Ende, die sich durch das Ausmaß der aufgebrachten weichlötbaren Beschichtung 13a unterscheiden. Während bei der links oben dargestellten Ausführung lediglich der Boden des Nagelkopfes und somit die endseitige Stirnfläche des Anschlussstiftes eine Beschichtung 13a trägt, erstreckt sich diese bei der rechts hiervon dargestellten Variante bis über den Umfang des nagelkopf-artigen verdickten Endabschnittes und einseitig bis über dessen der Stirnseite gegenüberliegende Deckfläche. Bei der Konfiguration rechts oben erstreckt sich die Beschichtung 13a hingegen nur bis zu einer vorbestimmten Höhe auf die Umfangsfläche des Nagelkopfes, bei der Konfiguration links unten über alle Flächen des Nagelkopfes und bei der Konfiguration rechts unten sogar ein Stück in die unbearbeitete Längserstreckung des Anschlussstiftes hinein.

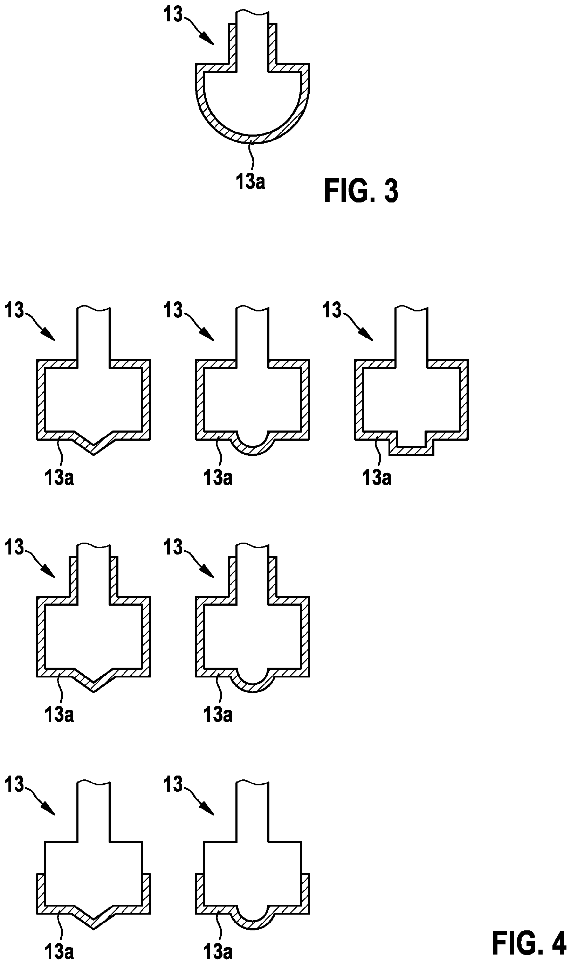

Fig. 3 zeigt einen weiteren Anschlussstift mit nagelkopf-artiger Verdickung des äußersten Endes, die hier jedoch halbkugelförmig ausgeführt ist. Diese Konfiguration lässt sich unter Einsatz eines entsprechenden Formwerkzeugs ausbilden.

Fig. 4 zeigt, in Anlehnung an Fig. 3, mehrere Ausgestaltungen eines nagelkopf-artig endseitig verdickten Anschlussstiftes 13, wobei die Ausführungen in der linken Spalte durch eine aus dem Boden des Nagelkopfes vorstehende kegelförmige Spitze, die Ausführungen in der mittleren Spalte durch eine aus dem Boden des Nagelkopfes hervorstehende halbkugelige Ausformungen und die Ausführung rechts oben durch eine aus dem Boden des Nagelkopfes zylindrisch hervorstehende Ausformung gekennzeichnet sind. Es sind verschiedene Beschichtungsvarianten gezeigt, die unter Bezugnahme auf Fig. 3 selbsterklärend sind und daher hier nicht weiter beschrieben werden.

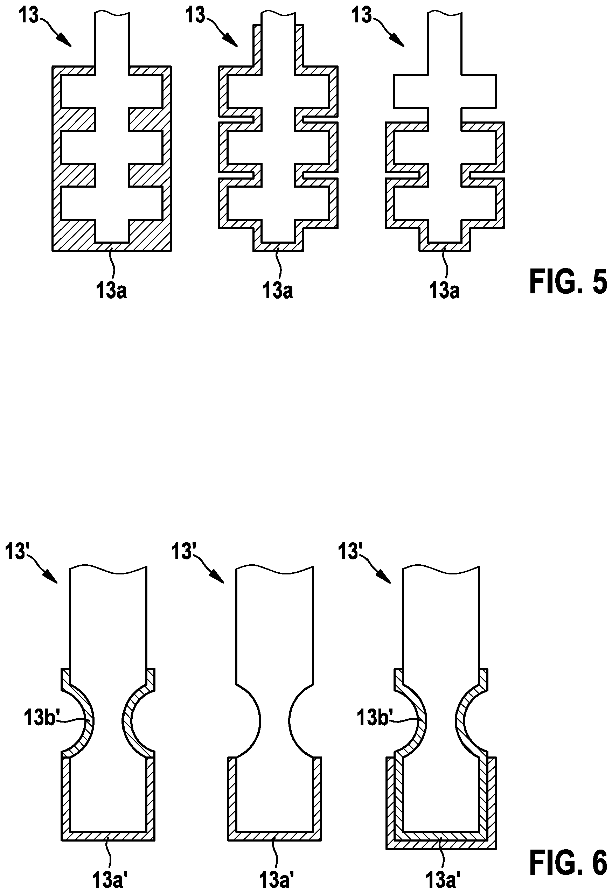

Fig. 5 zeigt drei Beschichtungsvarianten einer weiteren Ausführung des Anschlussstiftes 13, bei der aufeinanderfolgend drei scheibenförmige Verdickungen vorgesehen sind. In der Ausführung ganz unten sind nur das Stiftende und zwei der Verdickungen mit einer zusammenhängenden Beschichtung 13a versehen, während die Beschichtung sich bei der mittleren Variante über alle drei Verdickungen und ein Stück in den sich anschließenden Anschlussstift-Verlauf mit geringerem Durchmesser hinein erstreckt. Die oberste Ausführung unterscheidet sich von den anderen dadurch, dass die Beschichtung 13a hier in einer Weise gebildet ist, dass sie das Ende des Anschlussstiftes 13 in Art eines Zylinders umschließt, also zwischen den scheibenförmigen Verdickungen keine ringförmigen Rillen aufweist, wie sie bei den anderen beiden Ausführungen auftreten.

Fig. 6 zeigt drei Beschichtungsvarianten eines Anschlussstiftes 13', bei dem im Unterschied zu den Anschlussstiften 13 gemäß Fig. 2 bis 5 im Endabschnitt keine Verdickung gebildet ist, sondern mit Abstand vom Stiftende ein Abschnitt reduzierten Durchmessers vorhanden ist. Jenem gegenüber stellt sich das direkte Stiftende dann wiederum als Verdickung im Sinne der allgemeinen Ausführungen weiter oben dar. Bei der Konfiguration links in der Figur ist der Bereich reduzierten Durchmessers mit einer Diffusionsbarriereschicht 13b' bedeckt, während der Endabschnitt des Stiftes, einschließlich der endseitigen Stirnfläche, mit einer weichlötbaren Beschichtung 13a' versehen ist. Bei der mittleren Ausführung ist lediglich die weichlötbare Beschichtung 13a' des Endabschnitts vorgesehen, während der endfernere Abschnitt geringeren Durchmessers unbeschichtet ist. Bei der rechts dargestellten Konfiguration ist das gesamte Stiftende, also sowohl der Abschnitt reduzierten Durchmessers als auch der Endabschnitt und die Stirnfläche, zunächst mit einer diffusionshemmenden Beschichtung 13b' versehen, und im verdickten Bereich des Stiftendes ist jene erste Schicht noch mit einer zweiten Schicht (Deckschicht) 13' überdeckt, welche weichlötbar ist.

Die Ausführung der Erfindung ist in vielerlei anderen Ausführungen und in verschiedensten Kombinationen der oben als Merkmale verschiedener Ausführungsformen dargestellten Merkmale möglich, insbesondere auch mit mehr als zweiteiligen bzw. zweischichtigen Beschichtungssystemen, mit anderen als den oben genannten Materialien und in vielerlei geometrischen Ausgestaltungen der jeweiligen Verdickungen bzw. endferneren Bereiche geringeren Durchmessers.

## Patentansprüche

1. Anschlussstift (13; 13') zum elektrischen Anschluss eines Leitungsträgers (7), wobei der Anschlussstift zur stoffschlüssigen Anbindung an eine Leiterfläche des Leitungsträgers vorgesehen ist, mindestens abschnittsweise aus einem, insbesondere biokompatiblen, Nichtedelmetall gebildet ist
**dadurch gekennzeichnet, dass** der Anschlussstift (13; 13') an oder nahe einem Ende einen gegenüber einem end-ferneren Abschnitt verdickten Abschnitt hat und mindestens ein Teil der Oberfläche des verdickten Abschnitts eine weichlötbare Beschichtung (13a; 13a') aufweist.

2. Anschlussstift nach Anspruch 1, wobei der verdickte Abschnitt am Ende des Anschlussstiftes rotationssymmetrisch oder als vier- oder mehreckiges Prisma oder vier- oder mehrseitiger Pyramidenstumpf im Wesentlichen in Art eines Nagelkopfes gebildet ist.

3. Anschlussstift nach Anspruch 2, wobei am Boden des Nagelkopfes, mithin am äußersten Ende des Anschlussstiftes, ein insbesondere kegelförmiger, halbkugelförmiger, zylindrischer oder pyramidenförmiger Spitzenabschnitt ausgeformt ist.

4. Anschlussstift nach einem der vorangehenden Ansprüche, wobei der verdickte Abschnitt bezüglich eines end-ferneren Abschnitts gegeben ist, welcher einen gegenüber dem Durchmesser, den der Anschlussstift im größeren Teil seiner Längserstreckung hat, verringerten Durchmesser hat.

5. Anschlussstift nach Anspruch 4, wobei der, auf den end-ferneren Abschnitt mit verringertem Durchmesser bezogen, verdickte Abschnitt den gleichen Durchmesser wie der Anschlussstift im größeren Teil seiner Längserstreckung aufweist.

6. Anschlussstift nach Anspruch 2 oder 3 und Anspruch 4, wobei der end-fernere Abschnitt mit verringertem Durchmesser unmittelbar benachbart oder mit vorgegebenem Abstand zum nagelkopf-artigen Endabschnitt des Anschlussstiftes gebildet ist.

7. Anschlussstift nach einem der vorangehenden Ansprüche, welcher mehrere verdickte und beschichtete Abschnitte aufweist.

8. Anschlussstift nach Anspruch 7, wobei mindestens ein Teil der mehreren verdickten und beschichteten Abschnitte mit einer zusammenhängenden Beschichtung (13a) versehen ist, die sich über dazwischenliegende Abschnitte geringeren Durchmessers erstreckt.

9. Anschlussstift nach einem der vorangehenden Ansprüche, wobei die Beschichtung (13a; 13a') die gesamte Oberfläche des oder jeden verdickten Abschnitts bedeckt.

10. Anschlussstift nach einem der Ansprüche 2 bis 8, wobei die Beschichtung (13a; 13a') nur den Boden und optional den Umfang oder Teilabschnitte des Umfangs des nagelkopf-artigen verdickten Abschnitts bedeckt.

11. Anschlussstift nach einem der vorangehenden Ansprüche, wobei die Beschichtung (13a; 13a') mindestens eines der Metalle Gold, Platin, Kupfer, Silber, Nickel, Iridium und Palladium aufweist.

12. Anschlussstift nach einem der vorangehenden Ansprüche, wobei die Beschichtung (13a) mehrschichtig ist und insbesondere eine Deckschicht mindestens eines der Metalle Gold, Platin, Kupfer, Silber, Nickel, Iridium und Palladium aufweist.

13. Anschlussstift nach Anspruch 12, wobei eine direkt das unedle Metall des Anschlussstifts berührende Schicht (13b') der Beschichtung als Diffusionsbarriere ausgebildet ist und insbesondere mindestens eines der Metalle Aluminium, Nickel, Molybdän oder Gold aufweist.

14. Anschlussstift nach einem der vorangehenden Ansprüche, wobei die Beschichtung (13a; 13a') oder mindestens eine Schicht hiervon als galvanische Beschichtung oder als durch ein Vakuum-Beschichtungsverfahren erzeugte Dünnschicht ausgeführt ist.

15. Anschlussstift nach einem der vorangehenden Ansprüche, mit mindestens einem Abschnitt, der eine Beschichtung mit einem biokompatiblen Material aufweist, wie Titan, Tantal, Niob, Gold, Platin, Palladium oder einer Legierung mindestens eines dieser Metalle.

16. Durchführung (11) eines implantierbaren elektromedizinischen Gerätes (1), insbesondere eines Herzschrittmachers oder Kardioverters, die mindestens einen Anschlussstift (13a; 13a') nach einem der vorangehenden Ansprüche umfasst.

## Claims

1. A terminal pin (13; 13') for electrically connecting a conductor support (7), the terminal pin being provided for integral joining to a conductor surface of the conductor support, and formed at least in sections of an, in particular, biocompatible base metal, **characterized in that** the terminal pin (13; 13') at or near one end comprises a section that is thickened relative to a section located further from the end and at least a portion of the surface of the thickened section comprises a soft-solderable coating (13a; 13a').

2. The terminal pin according to claim 1, wherein the thickened section at the end of the terminal pin is rotation-symmetrical or formed as a quadrangular or polygonal prism or a four-sided or multi-sided truncated pyramid, substantially in the manner of nail head.

3. The terminal pin according to claim 2, wherein an, in particular, conical, hemispherical, cylindrical or pyramidal tip section is formed at the base of the nail head, and thus at the outermost end of the terminal pin.

4. The terminal pin according to any one of the preceding claims, wherein the thickened section is defined relative to a section that is located further from the end and that has a diameter that is reduced compared to the diameter of the terminal pin in the larger portion of the longitudinal extension thereof.

5. The terminal pin according to claim 4, wherein the thickened section, relative to the section that is located further from the end and has the reduced diameter, has the same diameter as the terminal pin in the larger portion of the longitudinal extension thereof.

6. The terminal pin according to claim 2 or 3 and claim 4, wherein the section that is located further from the end and has the reduced diameter is formed directly adjacent to or at a predefined distance from the nail head-shaped end section of the terminal pin.

7. The terminal pin according to any one of the preceding claims, comprising a plurality of thickened and coated sections.

8. The terminal pin according to claim 7, wherein at least some of the plurality of thickened and coated sections are provided with a contiguous coating (13a), which extends across interposed sections having smaller diameters.

9. The terminal pin according to any one of the preceding claims, wherein the coating (13a; 13a') covers the entire surface of the, or each, thickened section.

10. The terminal pin according to any one of claims 2 to 8, wherein the coating (13a; 13a') covers only the base, and optionally the circumference or partial sections of the circumference, of the nail head-shaped thickened section.

11. The terminal pin according to any one of the preceding claims, wherein the coating (13a; 13a') comprises at least one of the metals gold, platinum, copper, silver, nickel, iridium and palladium.

12. The terminal pin according to any one of the preceding claims, wherein the coating (13a) comprises multiple layers and, in particular, comprises a cover layer made of at least one of the metals gold, platinum, copper, silver, nickel, iridium and palladium.

13. The terminal pin according to claim 12, wherein a layer (13b') of the coating which makes direct contact with the base metal of the terminal pin is designed as a diffusion barrier and, in particular, comprises at least one of the metals aluminum, nickel, molybdenum or gold.

14. The terminal pin according to any one of the preceding claims, wherein the coating (13a; 13a'), or at least one layer thereof, is designed as a galvanic coating or as a thin film generated by way of a vacuum coating process.

15. The terminal pin according to any one of the preceding claims, comprising at least one section that includes a coating made of a biocompatible material, such as titanium, tantalum, niobium, gold, platinum, palladium or an alloy of at least one of these metals.

16. A feedthrough (11) of an implantable electromedical device (1), and in particular of a cardiac pacemaker or cardioverter, comprising at least one terminal pin (13a; 13a') according to any one of the preceding claims.

## Revendications

1. Broche de raccordement (13; 13') pour le raccordement électrique d'un porte-conducteur (7), où la broche de raccordement est prévue sur une surface conductrice du porte-conducteur pour une connexion par complémentarité des matières, constituée au moins par endroits d'un métal non noble, en particulier, biocompatible, **caractérisée en ce que** la broche de raccordement (13 ; 13') a un segment épaissi sur ou proche d'une extrémité par rapport à un segment plus éloigné et au moins une partie de la surface du segment épaissi présente un revêtement (13a ; 13a') pouvant être brasé de manière tendre.

2. Broche de raccordement selon la revendication 1, dans laquelle le segment épaissi à l'extrémité de la broche de raccordement est conçu de manière symétrique par rotation ou sous la forme d'un prisme à quatre ou plusieurs faces ou sous la forme d'un tronc de pyramide à quatre ou plusieurs faces, essentiellement à la manière d'une pointe de clou.

3. Broche de raccordement selon la revendication 2, dans laquelle, au niveau de la base de la pointe de clou, donc au niveau de l'extrémité la plus à l'extérieur de la broche de raccordement, il est formé un segment en pointe notamment en forme de quille, en forme de demie sphère, en forme cylindrique ou pyramidale.

4. Broche de raccordement selon l'une des revendications précédentes, dans laquelle le segment épaissi est référencé par rapport à un segment plus éloigné de l'extrémité, lequel a un diamètre réduit par rapport au diamètre que possède la broche de raccordement dans la majeure partie de son extension longitudinale.

5. Broche de raccordement selon la revendication 4, dans laquelle le segment épaissi, par rapport au segment le plus éloigné de l'extrémité avec un diamètre réduit, présente le même diamètre que la broche de raccordement dans la majeure partie de son extension longitudinale.

6. Broche de raccordement selon la revendication 2 ou 3, et la revendication 4, dans laquelle le segment le plus éloigné de l'extrémité avec un diamètre réduit est conçu immédiatement voisin, ou avec une distance prédéfinie par rapport, au segment d'extrémité à la manière d'une pointe de clou de la broche de raccordement.

7. Broche de raccordement selon l'une des revendications précédentes, laquelle présente plusieurs segments épaissis et revêtus.

8. Broche de raccordement selon la revendication 7, dans laquelle au moins une partie des segments épaissis et revêtus est munie d'un revêtement (13a) continu qui s'étend sur des segments intermédiaires de diamètre inférieur.

9. Broche de raccordement selon l'une des revendications précédentes, dans laquelle le revêtement (13a ; 13a') recouvre la surface totale du ou de chaque segment épaissi.

10. Broche de raccordement selon l'une des revendications 2 à 8, dans laquelle le revêtement (13a ; 13a') recouvre uniquement la base et éventuellement le pourtour, ou des segments partiels du pourtour, du segment épaissi à la manière d'une pointe de clou.

11. Broche de raccordement selon l'une des revendications précédentes, dans laquelle le revêtement (13a ; 13a') présente au moins l'un des métaux parmi l'or, le platine, le cuivre, l'argent, le nickel, l'iridium et le palladium.

12. Broche de raccordement selon l'une des revendications précédentes, dans laquelle le revêtement (13a) est multicouche et, en particulier, une couche de couverture présente au moins l'un des métaux parmi l'or, le platine, le cuivre, l'argent, le nickel, l'iridium et le palladium.

13. Broche de raccordement selon la revendication 12, dans laquelle une couche (13b') du revêtement touchant directement le métal non noble de la broche de raccordement est conçue sous la forme d'une barrière de diffusion et présente notamment au moins l'un des métaux parmi l'aluminium, le nickel, le molybdène ou l'or.

14. Broche de raccordement selon l'une des revendications précédentes, dans laquelle le revêtement (13a; 13a'), ou au moins une couche de celui-ci, est conçu sous la forme d'un revêtement galvanique ou d'une couche mince générée par un procédé de revêtement sous vide.

15. Broche de raccordement selon l'une des revendications précédentes, avec au moins un segment qui présente un revêtement avec un matériau biocompatible comme le titane, le tantale, le niobium, l'or, le platine, le palladium ou un alliage d'au moins un de ces métaux.

16. Traversée (11) d'un appareil électromédical (1) implantable, notamment d'un stimulateur cardiaque, ou d'un cardioverteur, qui comprend au moins une broche de raccordement (13a ; 13a') selon l'une des revendications précédentes.
